# EUROPEAN PATENT SPECIFICATION

(11) **EP 1 611 117 B1**
(45) Date of publication and mention of the grant of the patent: **14.09.2011**
(21) Application number: 04715069.3
(22) Date of filing: 26.02.2004
(51) Int. Cl.: C07D 301/10

(54) **A METHOD OF MANUFACTURING ETHYLENE OXIDE**
VERFAHREN ZUR HERSTELLUNG VON ETHYLENOXID
PROCEDE DE FABRICATION D'OXYDE D'ETHYLENE

(30) Priority: 28.02.2003 US 450848 P
(43) Date of publication of application: 04.01.2006
(73) Proprietor: Shell Internationale Research Maatschappij B.V., 2596 HR Den Haag (NL)
(72) Inventor: EVANS, Wayne, Errol, Richmond, Texas 77469 (US); MATUSZ, Marek, Houston, Texas 77084 (US); TE RAA, Arend Jan CRI/Criterion, 2514 AB Den Haag (NL)
(74) Representative: Matthezing, Robert Maarten
(86) International application number: PCT/US2004/005774
(87) International publication number: WO 2004/078737

(56) References cited:
- EP-A- 0 393 785
- EP-A- 0 480 539
- US-A- 4 822 900
- US-A- 4 831 162

## Description

This invention relates to the efficient manufacture of ethylene oxide by the partial oxidation of ethylene with oxygen using a high selectivity epoxidation catalyst.

In recent years new highly selective ethylene epoxidation catalysts have been developed that provide for selectivity benefits over conventional high activity epoxidation catalysts. Such high selectivity catalysts are known from U.S. Patents 4,761,394 and 4,766,105. However, the high selectivity catalysts employ higher reaction temperatures than do the high activity catalysts for a given ethylene oxide yield, and they exhibit a greater rate of catalyst deactivation than conventional high activity epoxidation catalysts.

It is, therefore, desirable to find a way to take advantage of the selectivity benefits from using a high selectivity epoxidation catalyst in the manufacture of ethylene oxide without incurring such associated negative effects as indicated hereinbefore.

Under commercial operation of epoxidation processes, the epoxidation reactor feed is formed by adding fresh oxygen and ethylene to a recycle gas stream which comprises, besides unreacted and recycled oxygen and ethylene, quantities of carbon dioxide, water, and other gases.

In accordance with the invention, provided is a method of manufacturing ethylene oxide, said method comprising contacting a high selectivity epoxidation catalyst under epoxidation reaction conditions at a reaction temperature below 260 °C with a reactor feed comprising ethylene, oxygen, and a concentration of carbon dioxide, wherein said concentration of carbon dioxide is less than 2 mole percent based on the total epoxidation reactor feed.

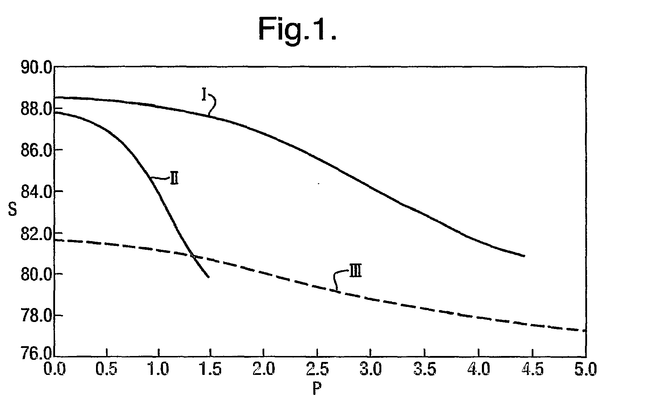

FIG. 1 demonstrates the improvement in catalytic life and selectivity of a high selectivity catalyst with plots of catalyst selectivity ("S", in %), at a given work rate, as a function of cumulative ethylene oxide production ("P", in kton/m³) for the inventive use of a high selectivity epoxidation catalyst ("I") as compared to the conventional use of a high selectivity epoxidation catalyst ("II") and the conventional use of a high activity catalyst ("III");

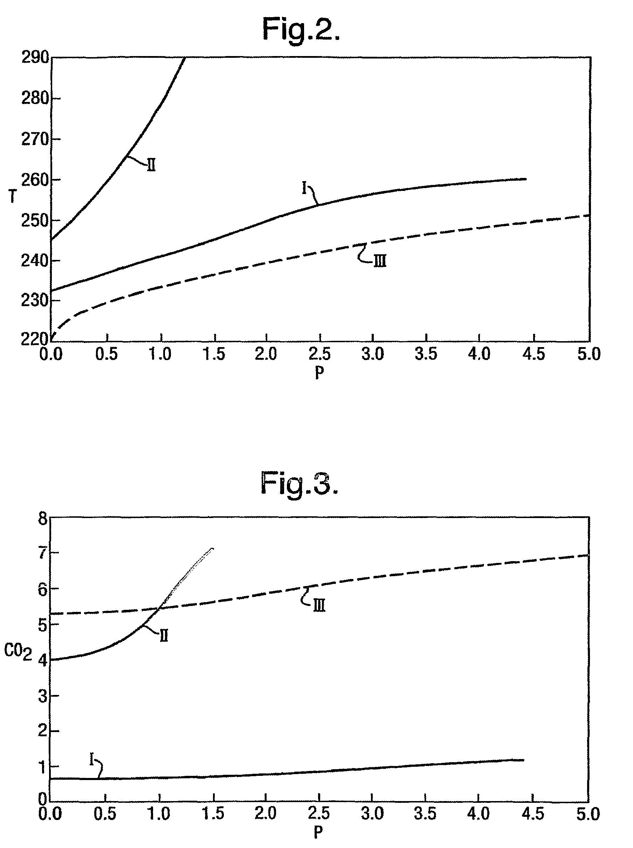

FIG. 2 demonstrates the improvement in catalytic life and reaction temperature with plots of reactor coolant temperature ("T", in °C) as a function of cumulative ethylene oxide production ("P", in kton/m³) for the inventive use of a high selectivity epoxidation catalyst ("I") and as compared to the conventional use of high selectivity epoxidation catalyst ("II") and the conventional use of a high activity catalyst ("III"); and

FIG. 3 presents plots of the reactor inlet concentration of carbon dioxide ("CO₂", in mole %) as a function of the cumulative ethylene oxide production ("P", in kton/m³) corresponding to the values for selectivity and reactor coolant temperature presented in FIG. 1 and FIG. 2.

It has been discovered that in the use of high selectivity epoxidation catalysts in the manufacture of ethylene oxide by the partial oxidation of ethylene with oxygen at constant conversion or work rate great improvement in the useful life of the catalyst and other benefits are obtainable by altering the composition of the typical epoxidation reactor feed. In an ethylene oxide process a typical epoxidation reactor feed generally comprises ethylene, oxygen and a concentration of carbon dioxide with the concentration in most cases exceeding 4 mole percent of the total moles of epoxidation reactor feed. This high carbon dioxide concentration in the epoxidation reactor feed does not normally have a significantly undesirable impact on the catalytic performance characteristics of high activity catalysts; however, with the use of a high selectivity epoxidation catalysts, as opposed to the use of a high activity epoxidation catalyst, in an epoxidation process huge process benefits are obtainable with the processing of an epoxidation reactor feed that has a carbon dioxide concentration that is less than 1 mole percent and even less than 2 mole percent of the total reactor feed.

As used herein, the term "selectivity" means the mole percent of the desired ethylene oxide formed relative to the total ethylene converted at a given work rate. The term "work rate" for a given catalyst is defined to mean the amount of ethylene oxide produced per unit volume of catalyst (e.g., kg per m³) per hour. As it is used herein with reference to the activity of a catalyst, the term "activity" means the temperature required by a catalyst to provide for a given work rate.

Thus, a high activity epoxidation catalyst is a catalyst that employs a lower reaction temperature for a given ethylene oxide yield per quantity of epoxidation catalyst when compared to an alternative epoxidation catalyst. And, a high selectivity epoxidation catalyst is a catalyst, that for a given temperature, provides for a greater percentage of a converted feed that is converted to ethylene oxide product than an alternative epoxidation catalyst.

Both the high activity catalyst and the high selectivity catalyst that are referred to herein are supported silver-based catalysts, but the two catalysts have different catalytic performance characteristics.

The material of the supported silver-based catalysts can be selected from a wide range of porous support materials particularly those which are considered to be inert in the presence of the ethylene oxidation feeds, products and reaction conditions. Such materials can be natural or artificial, and they can include the aluminum oxides, magnesia, zirconia, silica, silicon carbide, clays, pumice, zeolites and charcoal. Alpha alumina is a preferred material for use as the main ingredient of the porous support.

The support material is porous and preferably has a surface area, as measured by the B.E.T. method, of less than 20 m²/g and more in particular from 0.05 to 20 m²/g. Preferably the B.E.T. surface area of the support is in the range of from 0.1 to 10, more preferably from 0.1 to 3.0 m²/g. The B.E.T. method of measuring the surface area has been described in detail by Brunauer, Emmet and Teller in J.Am.Chem.Soc. 60 (1938) 309-316.

The highly selective supported silver-based catalyst of the invention may be one which has an initial selectivity of at least 85%, preferably at least 86% and, most preferably, at least 87%. In comparison, the initial selectivity of highly active supported silver-based catalysts is less than the initial selectivity of the highly selective supported silver-based catalysts and, more specifically, the initial selectivity of the highly active supported silver-based catalysts may be less than 85%. It is recognized, however, that from a practical standpoint the highly active catalyst will have some minimum selectivity. This minimum selectivity value is believed to be no lower than 78%.

The term initial selectivity referred to herein means the selectivity of the given catalyst when it is fresh and unused. This recognizes that a catalyst can lose activity with use. The initial selectivity of a given catalyst is determined by measuring the selectivity of the catalyst using a standard testing procedure. In this standard testing procedure, a crushed catalyst (1.27-1.81 mm particle size, i.e. 14-20 mesh sieve fraction) is placed within the 6.35 mm (1/4 inch) diameter stainless steel U-tube of a micro-reactor operated under certain specified process conditions. A standard feed of 30 mole percent ethylene, 7 mole percent carbon dioxide, and 8.5 mole percent oxygen, and 54.5 mole percent nitrogen is introduced into the micro-reactor at a pressure of 1447 kPa gauge (210 psig) and at such a rate as to provide a gaseous hourly space velocity of 3300 Nl/(l.h). The selectivity, Sw, and activity, Tw, are determined for a work rate of 200 kg ethylene oxide yield per hour per cubic meter of catalyst. The selectivity is presented in terms of mole percent, and the activity is presented in terms of temperature in degrees centigrade.

In addition to the differences in the measured catalytic performances between the highly active and highly selective catalysts there also can be differences in the types and amounts of catalytic promoter compounds used in the two catalysts. One difference is that the high selectivity catalysts of the invention include a rhenium promoter component, whereas, on the other hand, the high activity catalysts contain, if any, an insignificant or nonpromoting amount of a rhenium component. Also, in addition to the rhenium component, the high selectivity catalysts can further contain a promoting amount of an alkali metal promoter or a further metal promoter, or both. Suitable high selectivity catalysts are described in detail in U.S. Patents 4,761,394 and 4,766,105, which are incorporated herein by reference.

The high selectivity catalysts, thus, may comprise a support material, a catalytically effective amount of silver, a promoting amount of rhenium and, optionally, a promoting amount of one or more alkali metals and, optionally, a promoting amount of one or more additional promoter metals. The amount of silver in the high selectivity catalyst can be in the range of from a catalytically effective amount upwardly to 40 percent by weight of the total catalyst. Preferably, the amount of silver can range up to 40 weight percent based on the total weight of the catalyst. More preferably, the amount of silver can range from 1 to 30 weight percent based on the total weight of the catalyst and, most preferably, from 5 to 20 weight percent.

The amount of rhenium in the high selectivity catalyst is a promoting amount generally ranging from a promoting amount upwardly to 20 micromoles of rhenium per gram of catalyst. The preferred amount of rhenium in the high selectivity catalyst ranges from 0.1 micromoles per gram to 10 micromoles per gram, more preferably from 0.2 micromoles per gram to 5 micromoles per gram of total catalyst, or, alternatively stated, from 19 parts per million to 1860 parts per million, preferably from 37 parts per million to 930 parts per million by weight of total catalyst.

The amount of alkali metal in the high selectivity catalyst, if any, is a promoting amount, generally ranging from a promoting amount upwardly to 4000 parts per million by weight of the total catalyst (ppmw). Preferably, the amount of alkali metal, when present, is in the range of from 10 to 3000 ppmw, more preferably, from 15 to 2000 ppmw and, even more preferably, from 20 to 1500 ppmw.

The optional additional metal promoter of the high selectivity catalyst can be selected from the group of metals consisting of sulfur, molybdenum, tungsten, chromium, and mixtures of two or more thereof. The amount of additional metal promoters in the high selectivity catalyst, if any, is generally in the range of from 0.1 to 10 millimoles per kilogram of total catalyst, and, preferably, from 0.2 to 5 millimoles per kilogram of total catalyst.

As for the high activity catalyst, in addition to it being different from the high selectivity catalyst by exhibiting a lower selectivity as described above, it ordinarily does not contain a rhenium promoter, but it can contain an alkali metal promoter. Thus, the high activity catalyst preferably can comprise a support material, a catalytically effective amount of silver and a promoting amount of alkali metal but excluding a promoting amount of rhenium. Thus, the high activity catalyst can also consist essentially of a catalytically effective amount of silver, a promoting amount of alkali metal and a support material. Examples of suitable high activity catalysts are described in U.S. Patent 5,380,697, which is incorporated herein by reference.

The silver component can be present in the high activity catalyst in the range of from a catalytically effective amount to 40 weight percent based on the total weight of the catalyst. Preferably, however, the silver is present in the range of from 1 to 30 weight percent and, most preferably, from 5 to 20 weight percent.

The alkali metal component can be present in the high activity catalyst in the range of from a promoting amount upwardly to 4000 ppmw. Preferably, the alkali metal is present in the range of from 10 to 3000 ppmw and, more preferably, from 15 to 2000 ppmw.

The inventive method includes contacting, under suitable epoxidation reaction conditions, an epoxidation reactor feed with a high selectivity epoxidation catalyst as defined herein. The high selectivity epoxidation catalyst, in most instances, is contained within an epoxidation reaction zone defined by an epoxidation reactor that provides means for contacting the epoxidation reactor feed with the high selectivity epoxidation catalyst under such suitable epoxidation reaction conditions.

The epoxidation reactor feed of the inventive method comprises ethylene, oxygen and a concentration of carbon dioxide. The epoxidation reactor feed can also comprise additional other compounds, for example, argon, or nitrogen, or methane, or ethane, or some combination of such other compounds, and some water. Typically, the amount of ethylene in the epoxidation reactor feed can be in the range of from 1 to 40 mole percent, the amount of oxygen in the epoxidation reactor feed, excluding nitrogen, can be in the range of from 3 to 12 mole percent, the amount of other compounds in the epoxidation reactor feed can be in the range upwardly to 3 mole percent, with the values of mole percent being based on the total moles of the epoxidation reactor feed.

The epoxidation reactor feed contacted with the high selectivity catalyst has a low concentration of carbon dioxide which is generally well below 2 mole percent of the total epoxidation reactor feed. It is best for the concentration of carbon dioxide in the epoxidation reactor feed to be less than 1.5 mole percent of the total epoxidation reactor feed, but, preferably, the carbon dioxide concentration of the epoxidation reactor feed is maintained below 1.2 mole percent and, most preferably, the carbon dioxide concentration is below 1 mole percent. In accordance with this invention, the concentration of carbon dioxide in the epoxidation reactor feed is at least 0.1 mole percent, typically at least 0.2 mole percent of the total moles of epoxidation reactor feed.

As stated hereinbefore, under commercial operation of epoxidation processes, the epoxidation reactor feed is formed by adding fresh oxygen and ethylene to a recycle gas stream which comprises, besides unreacted and recycled oxygen and ethylene, quantities of water, carbon dioxide, and other gases. The water is normally introduced into the recycle gas stream in the carbon dioxide absorber of the commercial unit. It has been found that when practicing the present invention it is desirable that the water concentration of the epoxidation reactor feed is as low as possible, as this enables improving the catalyst performance in respect of activity, selectivity and catalyst life, relative to the situation where the epoxidation reactor feed comprises a relatively large amount of water. Preferably, the water concentration of the epoxidation reactor feed is at most 1.5 mole percent, more preferably at most 1 mole percent, most preferably at most 0.5 mole percent, and in particular at most 0.35 mole percent, of the total epoxidation reactor feed. In normal practice of this invention, the water concentration of the epoxidation reactor feed may typically be at least 0.01 mole percent, more typically at least 0.1 mole percent, of the total epoxidation reactor feed.

One of the features of the inventive method is that it provides for a catalyst selectivity exceeding 82 mole percent for an epoxidation reaction temperature that is below 260 °C, and it can even provide for a catalyst selectivity exceeding 85 mole percent for an epoxidation reaction temperature that is below 250 °C. Preferably, the inventive method provides for an epoxidation reaction temperature of less than 240 °C for a catalyst selectivity exceeding 88 mole percent.

Suitable epoxidation reaction conditions of the inventive method can include an epoxidation reaction zone temperature maintained during the contacting of the high selectivity epoxidation catalyst with the epoxidation reactor feed that is at least 180 °C, for example in the range of from 180 to 250 °C or from 180 to 240 °C; in particular at least 190 °C, for example in the range of from 190 to 250 °C or from 190 to 240 °C; and more in particular at least 200 °C, for example in the range of from 200 to 250 °C or from 200 to 240 °C. The gaseous hourly space velocity is generally in the range of from 1500 Nl/(l.h) to 10,000 Nl/(l.h), and the epoxidation reaction zone inlet pressure is generally in the range upwardly to 35 bar, preferably from 5 to 30 bar.

The ethylene oxide produced by the inventive method may be converted into 1,2-ethanediol, a 1,2-ethanediol ether, or an ethanolamine. As this invention leads to a more attractive process for the manufacture of ethylene oxide, it concurrently leads to a more attractive process which comprises producing ethylene oxide in accordance with the invention and the subsequent use of the obtained ethylene oxide in the manufacture of the 1,2-ethanediol, 1,2-ethanediol ether, and/or ethanolamine.

The conversion into 1,2-ethanediol or the 1,2-ethanediol ether may comprise, for example, reacting the ethylene oxide with water, suitably using an acidic or a basic catalyst. For example, for making predominantly 1,2-ethanediol and less 1,2-ethanediol ether, the ethylene oxide may be reacted with a ten fold molar excess of water, in a liquid phase reaction in presence of an acid catalyst, e.g. 0.5-1.0 %w sulfuric acid, based on the total reaction mixture, at 50-70 °C at 1 bar absolute, or in a gas phase reaction at 130-240 °C and 20-40 bar absolute, preferably in the absence of a catalyst. If the proportion of water is lowered the proportion of 1,2-ethanediol ethers in the reaction mixture is increased. The 1,2-ethanediol ethers thus produced may be a di-ether, triether, tetra-ether or a subsequent ether. Alternative 1,2-ethanediol ethers may be prepared by converting the ethylene oxide with an alcohol, in particular a primary alcohol, such as methanol or ethanol, by replacing at least a portion of the water by the alcohol.

The conversion into the ethanolamine may comprise, for example, reacting the ethylene oxide with ammonia. Anhydrous or aqueous ammonia may be used, although anhydrous ammonia is typically used to favour the production of monoethanolamine. For methods applicable in the conversion of the ethylene oxide into the ethanolamine, reference may be made to, for example US-A-4845296, which is incorporated herein by reference.

The 1,2-ethanediol and the 1,2-ethanediol ether may be used in a large variety of industrial applications, for example in the fields of food, beverages, tobacco, cosmetics, thermoplastic polymers, curable resin systems, detergents, heat transfer systems, etc. The ethanolamine may be used, for example, in the treating ("sweetening") of natural gas.

Unless specified otherwise, the low-molecular weight organic compounds mentioned herein, for example the 1,2-ethanediol ethers and reaction modifiers, have typically at most 40 carbon atoms, more typically at most 20 carbon atoms, in particular at most 10 carbon atoms, more in particular at most 6 carbon atoms. As defined herein, ranges for numbers of carbon atoms (i.e. carbon number) include the numbers specified for the limits of the ranges.

Having generally described the invention, a further understanding may be obtained by reference to the following examples, which are provided for purposes of illustration only and are not intended to be limiting unless otherwise specified.

### EXAMPLE 1

A high selectivity catalyst, containing silver and promoting amounts of rhenium, lithium, cesium and sulfur on alpha-alumina was tested in the production of ethylene oxide from ethylene and oxygen. To do this, a sample of crushed catalyst was loaded into a stainless steel U-shaped reactor tube. The tube was immersed in a molten metal bath (heat medium) at 180 °C, and the ends of the tube were connected to a gas flow system. A gas mixture passed through the catalyst bed, in a "once-through" operation. The weight of catalyst used and the inlet gas flow rate were adjusted to give a gas hourly space velocity of 3300 Nl/(l.h). The inlet gas pressure was 1550 kPa absolute.

The composition of the gas mixture was adjusted to 30 volume percent ethylene, 8 volume percent oxygen, 1 volume percent carbon dioxide, 2.5 parts per million by volume (ppmv) ethyl chloride, and nitrogen balance.

The temperature of the catalyst bed was ramped up at a rate of 10 °C per hour to 225 °C and then the temperature was adjusted so as to achieve an oxygen conversion of 40 mole percent. The ethyl chloride concentration in the gas mixture was adjusted to 2.5 ppmv so as to obtain an optimum selectivity of ethylene oxide formation. The activity of the catalyst is expressed as the temperature at which a 40 mole percent oxygen conversion is achieved (T40); the selectivity is the selectivity at the temperature T40. During the run the catalyst was subject to degradation, and in order to maintain a constant 40 mole percent oxygen conversion the temperature was gradually increased. The results are given in TABLE 1.

In three similar comparative tests, the concentration of carbon dioxide in the gas mixture was 5 to 7 percent volume, instead of 1 percent volume. The average result of the three comparative tests is also given in TABLE 1.

**TABLE 1**

| CO₂ concentration, %v | 1 | 5-7 |
|---|---|---|
| Run time, days | 263 | 195 |
| T40, initial, °C | 248 | 261 |
| Average activity decline rate, °C/month | 2.1 | 2.9 |
| Initial selectivity, %mole | 86.0 | 85.1 |
| Average selectivity decline rate, %mole/month | 0.7 | 1.1 |
| T40: temperature at 40 %mole oxygen conversion | | |

The results in TABLE 1 show clearly that a lower carbon dioxide concentration in the epoxidation reactor feed improves the performance of a high selectivity catalyst, in respect of its activity, selectivity and catalyst life.

### EXAMPLE 2

This calculated example presents data generated by a proprietary model for predicting the performance of a high selectivity epoxidation catalyst at the operating conditions of an hourly space velocity of 4700 Nl/(l.h), a pressure of 21.7 barg, and a work rate of 184 kg/(m³.h) for a reactor feed containing 25 mole percent ethylene and 8 mole percent oxygen. The model is based on the correlation of actual catalyst performance data gathered from numerous sources such as micro-reactor activity data, pilot plant data and other sources of catalyst performance data.

FIG. 1 presents the selectivity of a high selectivity epoxidation catalyst as a function of the age of the catalyst based on the cumulative ethylene oxide production in kton/m³ for the corresponding feedstock carbon dioxide concentrations presented in FIG. 3. The plots show that there is a strong relationship between catalyst life and feedstock carbon dioxide concentration and between selectivity and feedstock carbon dioxide concentration. As is shown in FIG. 1, the rate of decline in the selectivity of the catalyst when processing a feedstock having a carbon dioxide concentration of less than 1 mole percent (curve marked "I") is significantly lower than the rate of decline in the selectivity of the catalyst when processing a feedstock having a more conventional carbon dioxide concentration of greater than 4 mole percent (curve marked "II"). It is also noted that the initial selectivity of the high selectivity catalyst is higher for the case in which the feedstock has a carbon dioxide concentration of less than 1 mole percent as opposed to a feedstock carbon dioxide concentration of greater than 4 mole percent. These data demonstrate the great benefits in the selectivity and life of a high selectivity epoxidation catalyst that are obtainable from processing an epoxidation reactor feedstock having a low carbon dioxide concentration. Further comparative data relate to the use of a high activity catalyst operated at greater than 4 mole percent carbon dioxide concentration (curve marked "III").

FIG. 2 presents the reactor coolant temperature as a function of the age of the catalyst used in the epoxidation reaction for the corresponding feedstock carbon dioxide concentrations presented in FIG. 3. The reactor coolant temperature approximates the reaction temperature. As the data demonstrate, the epoxidation catalyst of the inventive method that processes an epoxidation reactor feedstock having a low carbon dioxide concentration of less than 1 mole percent (curve marked "I") loses its activity at significantly lower rate than the epoxidation catalyst of the conventional method that processes an epoxidation reactor feedstock having a significantly higher concentration of carbon dioxide than that of the inventive method (curve marked "II"). These data show that the stability of the high selectivity epoxidation catalyst in terms of the rate of decline in catalyst activity is significantly improved with the inventive method which includes the processing of an epoxidation feedstock having a very low carbon dioxide concentration. Further comparative data relate to the use of a high activity catalyst operated at greater than 4 mole percent carbon dioxide concentration (curve marked "III").

## Claims

1. A method of manufacturing ethylene oxide, said method comprises contacting a high selectivity supported silver-based epoxidation catalyst comprising a promoting amount of rhenium under epoxidation reaction conditions at a reaction temperature below 260 °C with a reactor feed comprising ethylene, oxygen, and a concentration of carbon dioxide, wherein said concentration of carbon dioxide is in the range of from 0.1 to less than 2 mole percent based on the total epoxidation reactor feed.

2. A method as recited in claim 1, wherein the temperature is below 250 °C, in particular in the range of from 190 to 250 °C.

3. A method as recited in claim 2, wherein the temperature is below 240 °C, in particular in the range of from 190 to 240 °C.

4. A method as recited in any of claims 1-3, wherein said concentration of carbon dioxide is in the range of from 0.1 to less than 1.5 mole percent, based on the total epoxidation reactor feed.

5. A method as recited in claim 4, wherein said concentration of carbon dioxide is in the range of from 0.2 to less than 1.2 mole percent, based on the total epoxidation reactor feed.

6. A method as recited in any of claims 1-5, wherein the water concentration of the epoxidation reactor feed is at most 1.5 mole percent of the total epoxidation reactor feed, in particular at most 1 mole percent of the total epoxidation reactor feed.

7. A method as recited in claim 6, wherein the water concentration of the epoxidation reactor feed is in the range of from 0.01 to 0.5 mole percent, in particular from 0.1 to 0.35 mole percent, of the total epoxidation reactor feed.

8. A method as recited in any of claims 1-7, wherein said high selectivity supported silver-based epoxidation catalyst comprises a support material, a catalytically effective amount of silver, and a promoting amount of rhenium.

9. A method as recited in claim 8, wherein the support material is an alpha alumina, the amount of silver is in the range of from 1 to 40 weight percent, and the amount of rhenium is in the range of from 0.1 to 10 micromoles per gram, based on the total weight of catalyst.

10. A method for making 1,2-ethanediol or a 1,2-ethanediol ether comprising:
- obtaining ethylene oxide by a method of manufacturing ethylene oxide as recited in any of claims 1-9, and
- converting ethylene oxide into 1,2-ethanediol or the 1,2-ethanediol ether.

## Patentansprüche

1. Verfahren zum Herstellen von Ethylenoxid, wobei das Verfahren umfasst: Inkontaktbringen eines hochselektiven, geträgerten, Silber basierenden Epoxidierungskatalysators umfassend eine Promotormenge an Rhenium unter Epoxidierungsreaktionsbedingungen bei einer Reaktionstemperatur von unter 260°C mit einem Reaktorzulauf umfassend Ethylen, Sauerstoff, und eine Konzentration an Kohlenstoffdioxid, wobei die Konzentration an Kohlenstoffdioxid in dem Bereich von 0,1 bis weniger als 2 Molprozent, basierend auf dem gesamten Epoxidierungsreaktorzulauf, liegt.

2. Verfahren wie zitiert in Anspruch 1, wobei die Temperatur unter 250°C liegt, insbesondere in dem Bereich von 190 bis 250°C.

3. Verfahren wie zitiert in Anspruch 2, wobei die Temperatur unter 240°C liegt, insbesondere in dem Bereich von 190 bis 240°C.

4. Verfahren wie zitiert in einem der Ansprüche 1 - 3, wobei die Konzentration an Kohlenstoffdioxid in dem Bereich von 0,1 bis weniger als 1,5 Molprozent, basierend auf dem gesamten Epoxidierungsreaktorzulauf, liegt.

5. Verfahren wie zitiert in Anspruch 4, wobei die Konzentration an Kohlenstoffdioxid in dem Bereich von 0,2 bis weniger als 1,2 Molprozent, basierend auf dem gesamten Epoxidierungsreaktorzulauf, liegt.

6. Verfahren wie zitiert in einem der Ansprüche 1 - 5, wobei die Wasserkonzentration des Epoxidierungsreaktorzulaufs bei höchstens 1,5 Molprozent des gesamten Epoxidierungsreaktorzulaufs, insbesondere bei höchstens 1 Molprozent des gesamten Epoxidierungsreaktorzulaufs liegt.

7. Verfahren wie zitiert in Anspruch 6, wobei die Wasserkonzentration des Epoxidierungsreaktorzulaufs in dem Bereich von 0,01 bis 0,5 Molprozent des gesamten Epoxidierungsreaktorzulaufs, insbesondere bei 0,1 bis 0,35 Molprozent des gesamten Epoxidierungsreaktorzulaufs liegt.

8. Verfahren wie zitiert in einem der Ansprüche 1 - 7, wobei der hochselektive, geträgerte, Silber basierende Epoxidierungskatalysator ein Trägermaterial, eine katalytisch effektive Menge an Silber und eine Promotormenge an Rhenium umfasst.

9. Verfahren wie zitiert in Anspruch 8, wobei das Trägermaterial ein alpha-Aluminiumoxid ist, die Menge an Silber in dem Bereich von 1 bis 40 Gewichtsprozent liegt, und die Menge an Rhenium in dem Bereich von 0,1 bis 10 Mikromolen pro Gramm liegt, basierend auf dem gesamten Gewicht des Katalysators.

10. Verfahren zum Herstellen von 1,2-Ethandiol oder eines 1,2-Ethandiolethers umfassend:
- Erhalten von Ethylenoxid durch ein Verfahren zum Herstellen von Ethylenoxid wie zitiert in einem der Ansprüche 1 - 9, und
- Konvertieren von Ethylenoxid in 1,2-Ethandiol oder den 1,2-Ethandiolether.

## Revendications

1. Procédé de fabrication d'oxyde d'éthylène, ledit procédé comprenant le contact d'un catalyseur d'époxydation hautement sélectif à base d'argent supporté comprenant une quantité activatrice de rhénium, dans des conditions de réaction d'époxydation à une température réactionnelle inférieure à 260 °C, avec une charge d'alimentation de réacteur comprenant de l'éthylène, de l'oxygène et une concentration de dioxyde de carbone, ladite concentration de dioxyde de carbone étant située dans la plage allant de 0,1 pour cent en mole à moins de 2 pour cent en mole sur la base de la charge d'alimentation totale de réacteur d'époxydation.

2. Procédé selon la revendication 1, dans lequel la température est inférieure à 250 °C, en particulier située dans la plage allant de 190 °C à 250 °C.

3. Procédé selon la revendication 2, dans lequel la température est inférieure à 240 °C, en particulier située dans la plage allant de 190 °C à 240 °C.

4. Procédé selon l'une quelconque des revendications 1 à 3, dans lequel ladite concentration de dioxyde de carbone est située dans la plage allant de 0,1 % en mole à moins de 1,5 % en mole, sur la base de la charge d'alimentation totale de réacteur d'époxydation.

5. Procédé selon la revendication 4, dans lequel ladite concentration de dioxyde de carbone est située dans la plage allant de 0,2 pour cent en mole à moins de 1,2 pour cent en mole, sur la base de la charge d'alimentation totale de réacteur d'époxydation.

6. Procédé selon l'une quelconque des revendications 1 à 5, dans lequel la concentration en eau dans la charge d'alimentation de réacteur d'époxydation est au plus de 1,5 pour cent en mole de la charge d'alimentation totale de réacteur d'époxydation, en particulier au plus de 1 pour cent en mole de la charge d'alimentation totale de réacteur d'époxydation.

7. Procédé selon la revendication 6, dans lequel la concentration en eau dans la charge d'alimentation de réacteur d'époxydation est située dans la plage allant de 0,01 pour cent en mole à 0,5 pour cent en mole, en particulier de 0,1 pour cent en mole à 0,35 pour cent en mole, de la charge d'alimentation totale de réacteur d'époxydation.

8. Procédé selon l'une quelconque des revendications 1 à 7, dans lequel ledit catalyseur d'époxydation hautement sélectif à base d'argent supporté comprend un matériau de support, une quantité d'argent efficace sur le plan catalytique et une quantité activatrice de rhénium.

9. Procédé selon la revendication 8, dans lequel le matériau de support est une alumine alpha, la quantité d'argent est située dans la plage allant de 1 pour cent en poids à 40 pour cent en poids, et la quantité de rhénium est située dans la plage allant de 0,1 micromole par gramme à 10 micromoles par gramme, sur la base du poids total du catalyseur.

10. Procédé de préparation de 1,2-éthanediol ou d'un éther de 1,2-éthanediol, comprenant :
- l'obtention d'oxyde d'éthylène par un procédé de fabrication d'oxyde d'éthylène selon l'une quelconque des revendications 1 à 9, et
- la conversion de l'oxyde d'éthylène en 1,2-éthanediol ou en éther de 1,2-éthanediol.
